# EUROPÄISCHE PATENTSCHRIFT

(11) **EP 0 351 720 B1**
(45) Veröffentlichungstag und Bekanntmachung des Hinweises auf die Patenterteilung: **24.08.1994**
(21) Anmeldenummer: 89112897.7
(22) Anmeldetag: 14.07.1989
(51) Int. Cl.: A61K 31/40, A61K 31/42, A61K 31/425, A61K 31/44, A61K 31/445, A61K 31/495, A61K 31/535

(54) **Verwendung substituierter 3,4-Dihydro-2H-benzopyrane als Heilmittel gegen obstruktive Funktionsstörungen der Lunge**
Use of substituted 3,4-dihydro-2H-benzopyrans as medicaments against obstructive disorders of the lung
Utilisation des 3,4-dihydro-2H-benzopyrannes substitués comme médicament contre des maladies obstructives du poumon

(30) Priorität: 19.07.1988 DE 3824446
(43) Veröffentlichungstag der Anmeldung: 24.01.1990
(60) Teilanmeldung: 93105146.0
(73) Patentinhaber: HOECHST AKTIENGESELLSCHAFT, 65926 Frankfurt am Main (DE)
(72) Erfinder: Englert, Heinrich Christian, Dr., D-6238 Hofheim am Taunus (DE); Klaus, Erik, Dr., D-6233 Kelkheim (Taunus) (DE); Mania, Dieter, Dr., D-6240 Königstein/Taunus (DE); Schölkens, Bernward, Dr., D-6233 Kelkheim (Taunus) (DE)

(56) Entgegenhaltungen:
- EP-A- 0 277 611

## Beschreibung

Die Erfindung betrifft die Verwendung von 3,4-Dihydro-2H-benzo[b]pyranen der Formel I 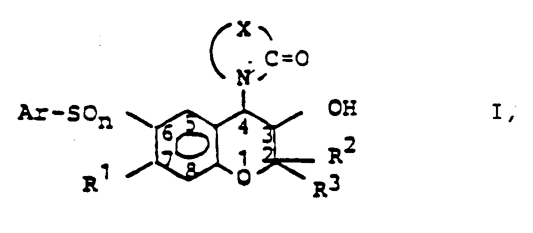
in welcher
- R¹: für H, OH, (C₁-C₂)-Alkoxy, (C₁-C₂)-Alkyl oder NR⁴R⁵ steht, wobei R⁴ und R⁵ gleich oder verschieden sind und für H, (C₁-C₂)Alkyl oder (C₁-C₃)-Alkylcarbonyl stehen,
- R²,R³: gleich oder verschieden sind und für Alkyl mit 1-4 C-Atomen stehen,
- Ar: für ein aromatisches oder heteroaromatisches System steht, das unsubstituiert oder durch 1 bis 3 gleiche oder verschiedene Reste (C₁-C₂)-Alkyl, (C₁-C₂)Alkoxy, Halogen, Trifluormethyl, CN, NO₂, CO-(C₁-C₂)Alkyl oder SOₘ-(C₁-C₂)-Alkyl mit m= 1 oder 2 substituiert ist,
- n: für 1 oder 2 steht,
- X: für eine Kette (CH₂)ᵣ steht, die durch ein Heteroatom 0, S oder NR⁶ unterbrochen sein kann, wobei R⁶ H oder (C₁-C₄)-Alkyl bedeutet und r für die Zahlen 2, 3, 4 oder 5 steht,
zur Herstellung eines Heilmittels gegen obstruktive Funktionsstörungen der Lunge.

Unter einem aromatischen System Ar wird vorzugsweise Phenyl, Naphthyl oder Biphenylyl verstanden, ein 5- oder 6-gliedriges heteroaromatisches System Ar ist vorzugsweise ein Rest eines 5- oder 6-gliedrigen O, N und/oder S-heterocyclischen Ringes, insbesondere Furyl, Thienyl, Isothiazolyl, Oxazolyl, Isoxazolyl, Pyrazolyl, Imidazolyl, Thiazolyl, Pyridyl, Pyrazinyl, Pyrimidinyl, Pyridazinyl, Triazinyl.

Unter Halogen wird F, Cl, Br oder J, vorzugsweise F und Cl verstanden.

Die C-Atome 3 und 4 des 3,4-Dihydro-2H-benzo[b]pyransystems (nachfolgend auch kurz "Chromansystem" genannt) der Formel I sind asymmetrisch substituiert. Gegenstand der Erfindung sind dabei nur solche Verbindungen, die an diesen Zentren entgegengesetzte Konfigurationen, also eine "trans"-Orientierung der Substituenten an diesen C-Atomen aufweisen. Enthält einer der Substituenten R¹, ArSOₙ, R² und/oder R³ Asymmetriezentren oder, falls R² und R³ ungleich sind (und somit ein asymmetrisches Kohlenstoffatom erzeugen), sind Verbindungen mit sowohl S- als auch R-konfigurierten Zentren Gegenstand der Erfindung.

Die Verbindungen können als optische Isomere, als Diastereoisomere, als Racemate oder als Gemische derselben vorliegen.

Bevorzugt werden Verbindungen der Formel I verwendet, bei denen R¹ bis R³, ArSOₙ die oben erwähnten Bedeutungen haben, X jedoch für eine Kette (CH₂)ᵣ steht mit r= 3 oder 4.

Ganz besonders bevorzugt verwendet werden solche Verbindungen, bei denen R¹ bis R³ die oben erwähnten Bedeutungen haben, Ar für Phenyl steht, das unsubstituiert oder wie oben definiert substituiert ist, n für 2 steht und X für eine Kette (CH₂)ᵣ mit r= 3 oder 4.

Insbesondere bevorzugte Verwendung finden solche Verbindungen, bei denen R¹ H bedeutet, R² und R³ für (C₁-C₂)Alkyl stehen, Ar für Phenyl steht, das unsubstituiert oder durch (C₁-C₂)-Alkyl, (C₁-C₂)-Alkoxy oder Halogen einfach substituiert ist, n für 2 steht und X für eine Kette (CH₂)ᵣ mit r= 3 oder 4.

Ebenfalls bevorzugt sind Verbindungen mit R¹ gleich H, R² und R³ gleich (C₁-C₂)-Alkyl, Ar gleich Phenyl, n gleich 2 und X gleich (CH₂)₃.

EP 0 176 689 beschreibt die Verwendung von Benzopyranen bei Atemwegskrankheiten und/oder Störungen des Gastrointestinaltrakts und/oder des Uterus, wobei insbesondere solche Störungen hervorgehoben werden, die bei glattmuskulären Kontraktionen auftreten.
EP 207 614 beschreibt die Verwendung von Benzopyranen bei der Inkontinenz. EP 277 611 beschreibt die Verwendung von Benzopyranen zur Herstellung eines Arzneimittels gegen Störungen der ableitenden Harnwege.
Ferner ist aus J. Med. Chem. 1986, 29, 2194 - 2201 bekannt, daß derartige Verbindungen blutdrucksenkende Eigenschaften haben können.
Überraschenderweise wurde nun für die Verbindungen I durch pharmakologische Untersuchungen gefunden, daß sie sich ebenfalls für eine Anwendung als Heilmittel gegen Atemwegskrankheiten.

Die Erfindung betrifft daher die Anwendung der Verbindungen der Formel I bei der Behandlung und Prophylaxe der vorstehend aufgeführten Krankheiten, besonders bevorzugt sind dabei solche Krankheiten, bei denen eine Störung der glattmuskulären Kontraktionen der jeweiligen Organe vorliegt wie beispielsweise Asthma. Eine besondere Bedeutung kommt dabei solchen Verbindungen I zu, deren blutdrucksenkende Eigenschaften weniger stark ausgeprägt sind.

Die Erfindung ist weiterhin gerichtet auf ein pharmazeutisches Präparat zur Behandlung von obstruktiven Funktionsstörungen der Lunge, dadurch gekennzeichnet, daß es eine Verbindung I als Wirkstoff neben üblichen Zuschlagstoffen enthält; sowie auf eine Verbindung I zur Anwendung zur Behandlung von obstruktiven Funktionsstörungen der Lunge.

Ganz besonders bevorzugt ist die Verwendung von 3,4-Dihydro-2,2-dimethyl-6-phenylsulfonyl-trans-4-(2-oxo-1-pyrrolidinyl)-2H-benzo[b]pyran-3-ol zur Herstellung eines Heilmittels gegen obstruktive Atemwegserkrankungen.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Die Verbindungen I können nach folgenden Verfahren hergestellt werden:
Indem man
a) Verbindungen der Formel II 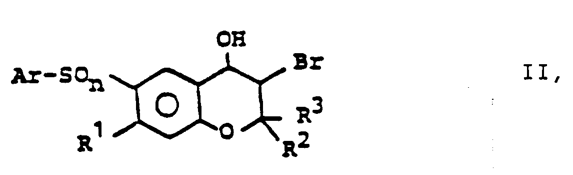 in denen R¹ bis R³ und ArSOₙ wie oben definiert sind umsetzt mit Lactamen der Formel III 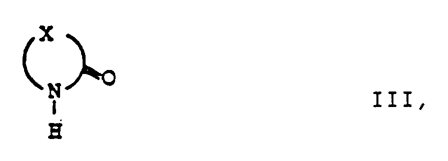
b) Verbindungen der Formel IV 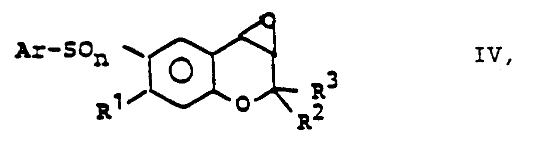 in denen R¹ bis R³ und ArSOₙ wie oben definiert sind, umsetzt mit den Lactamen der Formel III
c) Verbindungen der Formel V 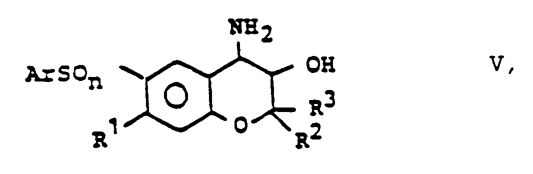 in denen R¹ bis R³ und ArSOₙ wie oben definiert sind, acyliert zu den Verbindungen VI 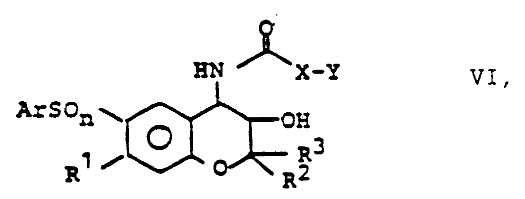 in denen Y eine Fluchtgruppe, wie etwa Chlor oder Brom, ist und R¹ bis R³ und ArSOₙ wie oben definiert sind, und diese zu den Verbindungen I cyclisiert
d) Verbindungen der Formel VII 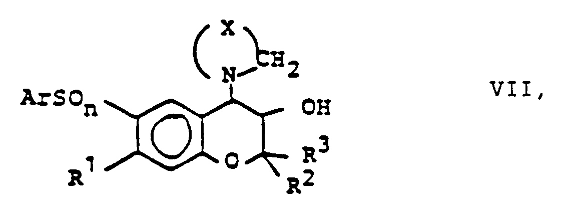 in denen R¹ bis R³ und ArSOₙ wie oben definiert sind, zu den Verbindungen I oxidiert.

Werden die Verbindungen I nach den Methoden a) oder b) hergestellt, so geschieht dies dadurch, daß man die Verbindungen II oder IV in einem geeigneten Lösemittel, vorzugsweise in dipolar aprotischen Lösemitteln wie etwa Dimethylsulfoxid oder THF umsetzt mit den Lactamen III, vorzugsweise unter Einwirkung von Basen, wie etwa Natriumhydrid, K-tert.butylat oder ähnlichen, für Lactam-N-Alkylierungen bekanntermaßen geeigneten Basen. Die Reaktionstemperatur ist dabei im weiten Bereich variierbar; vorzugsweise wird zwischen 0° und Zimmertemperatur gearbeitet oder bei Temperaturen, die leicht oberhalb der Zimmertemperatur liegen können.

Lactame der Formel III sind in vielen Fällen bekannt, oder können leicht nach literaturbekannten Methoden hergestellt werden. Verbindungen II oder IV sind neu. Sie können beispielsweise nach folgendem Syntheseweg hergestellt werden:
Verbindungen der Formel VIII 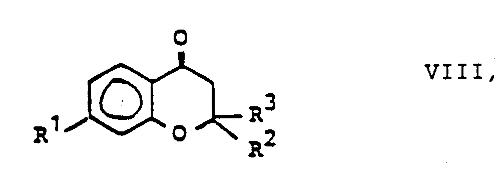
in denen R¹, R² und R³ wie oben definiert sind, werden mit Säurechloriden Ar-SOₙ-Cl in an sich bekannter Weise nach Art der Friedel-Crafts Acylierung umgesetzt zu Verbindungen der Formel IX 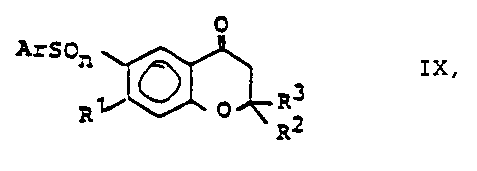
in denen R¹, R², R³ und Ar und n wie oben definiert sind. Diese werden durch Reduktionen unter Standardbedingungen etwa durch NaBH₄ in Methanol umgesetzt zu den Verbindungen X 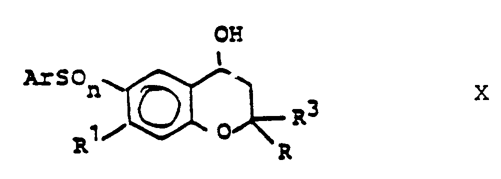
und anschließend einer Wasserabspaltung, etwa durch Pyridin/Phosphoroxychlorid, unterwerfen, wobei Verbindungen der Formel XI entstehen: 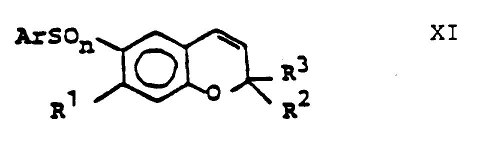
Verbindungen XI lassen sich nun leicht nach Standardmethoden in die Epoxide IV oder die Bromhydrine II umwandeln.

Steht bei dieser Reaktionssequenz R in der Bedeutung von NH₂ oder OH, so sind gegebenenfalls Schutzgruppen wie etwa die Dimethylaminomethylengruppe für NH₂ oder die Acetyl- oder Methylgruppe für die OH-Gruppe notwendig. Diese werden auf geeigneten Stufen, vorzugsweise nach Durchführung die in Verfahren a) oder b) beschriebenen Umsetzungen durch gängige Methoden wieder abgespaltet.

Chromene der Formel XI werden in einigen Fällen in an sich bekannter Weise durch thermisch induzierte Cyclisierung der entsprechenden Propargylether XII 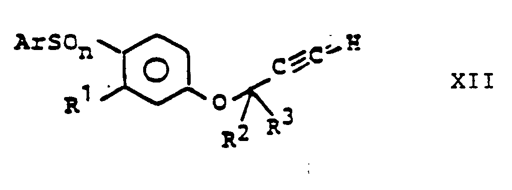
hergestellt. Diese wiederum lassen sich in an sich bekannter Weise aus den Phenolen XIII und den Propargylchloriden XIV herstellen. 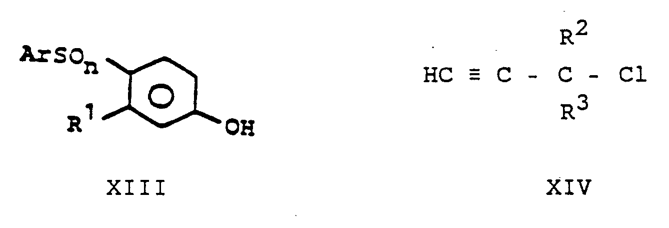
Die Verfahren c) und d) können dann besonders günstig angewandt werden, wenn enantiomerenreine Endprodukte I erwünscht sind. Verbindungen V und VII sind im Gegensatz zu Verbindungen I basisch und damit zur Salzbildung mit organischen Säuren befähigt. Durch Kristallisation mit einer geeigneten, optisch einheitlichen Säure wie etwa (+)-Mandelsäure oder (+)-Milchsäure können sie in an sich bekannter Weise enantiomerenrein erhalten werden und in enantiomerenreine Endprodukte I nach den Verfahren c) und d) umgewandelt werden.

Enantiomerenreine Endprodukte I können aber auch aus racemischen Endprodukten I durch gängige Racematspaltungsmethoden wie etwa die chromatographische Trennung unter Verwendung von chiralen Phasen oder die Derivatisierung der racemischen Produkte mit optisch einheitlichen Säurederivaten (Esterbildung über die 3-Hydroxygruppe des Chromansystems) oder mit optisch einheitlichen Isocyanaten (Carbamatbildung über die 3-Hydroxygruppe) erhalten werden. Die dabei erhaltenen diastereoisomeren Isocyanate oder Ester lassen sich durch gängige Methoden (Kristallisation oder Chromatographie) trennen und unter Abspaltung der optisch aktiven Hilfsgruppe an der 3-OH-Funktion in die optisch einheitlichen Endverbindungen I umwandeln. Als besonders vorteilhaft hat sich hierbei die Trennung der diastereomeren 3-Menthoxyacetate erwiesen.

Die Verbindungen I können wie bereits erwähnt erfindungsgemäß als Mittel zur Behandlung obstruktiver Atemwegskrankheiten eingesetzt werden. Arzneimittel, die die Verbindung I enthalten, können dabei oral, parenteral, intravenös, rektal oder durch Inhalation appliziert werden, wobei die bevorzugt Applikationsform von der zu behandelnden Krankheit abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffs der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindung ab; außerdem auch von Art und Stärke der zu behandelnden Krankheit sowie von Geschlecht, Alter, Gewicht und individueller Ansprechbarkeit des zu behandelndes Säugers. Im Durchschnitt beträgt die empfohlene tägliche Dosis einer Verbindung der Formel I bei einem etwa 75 kg schweren Patienten mindestens 0,1 mg, vorzugsweise mindestens 1 mg, bis maximal 100 mg, vorzugsweise bis maximal 10 mg. Bei akuten Ausbrüchen der Krankheit, etwa bei Asthmaanfällen, können dabei mehrere, z. B. bis zu 4 Einzeldosen pro Tag, erforderlich sein, während zur Prophylaxe auch eine Dosierung ausreichen kann.

Die Verbindungen I können dabei allein oder in Kombination mit anderen Verbindungen appliziert werden, etwa bei der Verwendung gegen obstruktive Atemwegsstörungen mit β₂-Agonisten wie Salbutamol oder mit Theophyllin oder mit Di-Na-Chromoglykat.

Die in der folgenden Tabelle zusammengestellten Verbindungen der Formel I sind besonders gut geeignet:
1) 2,2-Dimethyl-3,4-dihydro-7-methoxy-6-(p-chlorphenylsulfonyl)-trans-4-(2-oxo-1-pyrrolidinyl)-2H-benzo[b]pyran-3-ol,
2) 2,2-Dimethyl-3,4-dihydro-6-(p-chlorphenylsulfonyl)-trans-4-(2-oxo-1-piperidinyl)-2H-benzo[b]pyran-3-ol,
3) 2,2-Dimethyl-3,4-dihydro-6-(p-nitrophenylsulfonyl)-trans-4-(2-oxo-1-pyrrolidinyl)-2H-benzo[b]pyran-3-ol,
4) 2,2-Dimethyl-3,4-dihydro-6-(p-cyanophenylsulfonyl)-trans-4-(2-oxo-1-pyrrolidinyl)-2H-benzo[b]pyran-3-ol,
5) 2,2-Dimethyl-3,4-dihydro-6-(p-methoxyphenylsulfonyl)-trans-4-(2-oxo-1-pyrrolidinyl)-2H-benzo[b]pyran-3-ol,
6) 2,2-Dimethyl-3,4-dihydro-6-(p-trifluormethylphenylsulfonyl)-trans-4-(2-oxo-1-pyrrolidinyl)-2H-benzo[b]pyran-3-ol,
7) 2,2-Dimethyl-3,4-dihydro-6-(p-methylsulfonylphenylsulfonyl)-trans-4-(2-oxo-1-pyrrolidinyl)-2H-benzo[b]pyran-3-ol,
8) 2,2-Dimethyl-3,4-dihydro-6-(p-acetylphenylsulfonyl)-trans-4-(2-oxo-1-pyrrolidinyl)-2H-benzo[b]pyran-3-ol,
9) 2,2-Dimethyl-3,4-dihydro-7-methylamino-6-phenylsulfonyl-trans-4-(2-oxo-1-pyrrolidinyl)-2H-benzo[b]pyran-3-ol,
10) 2,2-Dimethyl-3,4-dihydro-7-fluor-6-phenylsulfonyl-trans-4-(2-oxo-1-pyrrolidinyl)-2H-benzo[b]pyran-3-ol,
11) 2,2-Diethyl-3,4-dihydro-7-fluor-6-phenylsulfonyl-trans-4-(2-oxo-1-pyrrolidinyl)-2H-benzo[b]pyran-3-ol,
12) 2,2-Dimethyl-7-chlor-3,4-dihydro-6-phenylsulfonyl-trans-4-(2-oxo-1-pyrrolidinyl)-2H-benzo[b pyran-3-ol,
13) 2,2-Dimethyl-3,4-dihydro-6-(4-chlor-3-methylphenylsulfonyl)-trans-4-(2-oxo-1-pyrrolidinyl)-2H-benzo[b]pyran-3-ol,
14) 2,2-Dimethyl-3,4-dihydro-6-(4-chlorphenylsulfonyl)-trans-(5-oxo-3-thiazolidinyl)-2H-benzo[b]pyran-3-ol,
15) 2,2-Dimethyl-3,4-dihydro-trans-4-(4-methyl-2-oxo-1-piperazinyl)-6-phenylsulfonyl-2H-benzo[b]pyran-3-ol,
16) 2,2-Dimethyl-3,4-dihydro-6-phenylsulfonyl-trans-4-(2-oxo-1-morpholinyl)-2H-benzo[b]pyran-3-ol,
17) 2,2-Dimethyl-3,4-dihydro-6-phenylsulfonyl-trans-4-(5-oxo-3-oxazolinyl)-2H-benzo[b]pyran-3-ol.
18) 3,4-Dihydro-2,2-dimethyl-6-(p-fluorphenylsulfonyl)-trans-4-(2-oxo-1-pyrrolidinyl)-2H-benzo[b]pyran-3-ol,
19) 3,4-Dihydro-2,2-dimethyl-6-(o-fluorphenylsulfonyl)-trans-4-(2-oxo-1-pyrrolidinyl)-2H-benzo[b]pyran-3-ol,
20) 3,4-Dihydro-2,2-dimethyl-6-(3-pyridylsulfonyl)-trans-4-(2-oxo-1-pyrrolidinyl)-2H-benzo[b]pyran-3-ol,
21) 3,4-Dihydro-2,2-dimethyl-6-(2-pyrimidinylsulfonyl)-trans-4-(2-oxo-1-pyrrolidinyl)-2H-benzo[b]pyran-3-ol,
22) 3,4-Dihydro-2,2-dimethyl-6-(2-furylsulfonyl)-trans-4-(2-oxo-1-pyrrolidinyl)-2H-benzo[b]pyran-3-ol.

### Beispiel 1

### 3,4-Dihydro-2,2-dimethyl-7-methoxy-6-(p-tolylsulfonyl)-trans-4-(2-oxo-1-pyrrolidinyl)-2H-benzo[b]pyran-3-ol

4,3 g (0,0097 Mol) 3-Brom-3,4-Dihydro-2,2-dimethyl-7-methoxy-6-(p-tolylsulfonyl)-2H-benzo[b]pyran-4-ol werden in 28 ml Dimethylsulfoxid gelöst, mit 3,5 ml 2-Pyrrolidinon (0,0465 Mol) und 0,78 g Natriumhydrid (80 %ige Suspension in Öl) (0,0325 Mol) versetzt und 3 Stunden bei 40°C gerührt. Man läßt über Nacht stehen, gießt den Ansatz auf Eiswasser, und saugt ab. Der Niederschlag wird aus Isopropanol umkristallisiert. Weiße Kristalle vom Schmp.: 263 - 65°C.

### Herstellung der Ausgangsverbindung:

3-Brom-3,4-dihydro-2,2-dimethyl-7-methoxy-6-(p-tolylsulfonyl)-2H-benzo[b]pyran-4-ol erhält man aus 2,2-Dimethyl-7-methoxy-6-(p-tolylsulfonyl)-2H-chromen und N-Bromsuccinimid in einem 9:1 Gemisch aus Dimethylsulfoxid/H₂O. Schmp.: 200-201°C

2,2-Dimethyl-7-methoxy-6-(p-tolylsulfonyl)-2H-chromen erhält man aus 2,2-Dimethyl-7-methoxy-6-(p-tolylsulfonyl)chroman-4-ol mit Phosphoroxychlorid/Pyridin in Benzol.
Schmp.: 132 - 33°C

2,2-Dimethyl-7-methoxy-6-(p-tolylsulfonyl)-chroman-4-ol erhält man aus 2,2-Dimethyl-7-methoxy-6-(p-tolylsulfonyl)chroman-4-on mit NaBH₄ in Ethanol.
Schmp.: 196 - 97°C

2,2-Dimethyl-7-methoxy-6-(p-tolylsulfonyl)chroman-4-on erhält man aus 2,2-Dimethyl-7-methoxy-chroman-4-on und p-Toluolsulfonsäurechlorid in Gegenwart von Aluminiumchlorid in Methylenchlorid.
Schmp.: 221 - 23°C.

### Beispiel 2

### 3,4-Dihydro-2,2-dimethyl-7-methoxy-6-(p-tolylsulfonyl)-trans-4-(2-oxo-1-piperidinyl)-2H-benzo[b]pyran-3-ol

5 g (0,011 Mol) 3-Brom-3,4-dihydro-2,2-dimethyl-7-methoxy-6-(p-tolylsulfonyl)-2H-benzo[b]pyran-4-ol werden in 32 ml Dimethylsulfoxid gelöst und mit 4,9 g Valerolactam (0,0526 Mol) sowie 0.8 g (0,033 Mol)NaH, 80 %ige Suspension in Öl, versetzt und 5 Stunden bei 40°C gerührt. Man gießt den Ansatz auf Eiswasser und saugt ab. Der Rückstand wird mehrfach mit Methanol ausgekocht.
Weiße Kristalle vom Schmp.: 261 - 63°C

### Beispiel 3

### 3,4-Dihydro-2,2-dimethyl-7-methoxy-6-phenylsulfonyl-trans-4-(2-oxo-1-pyrrolidinyl)-2H-benzo[b]pyran-3-ol

Die Verbindung wird analog Beispiel 1 aus 3-Brom-3,4-dihydro-2,2-dimethyl-7-methoxy-6-phenylsulfonyl-2H-benzo[b]pyran-4-ol hergestellt.
Weiße Kristalle vom Schmp.: 227 - 29°C

### Trennung der Antipoden, Beispiel 3a

1,075 g (0,0025 Mol) (±)-3,4-Dihydro-2,2-dimethyl-7-methoxy-6-phenylsulfonyl-trans-4-(2-oxo-1-pyrrolidinyl)-2H-benzo[b]pyran-3-ol werden in 5 ml 1,2-Dichlorbenzol gelöst, mit 0,9 g S(-)-1-Phenylethylisocyanat versetzt und ca 12 h bei 140°C gerührt. Der komplette Ansatz wird anschließend an Kieselgel mit dem Laufmittelsystem Toluol/Essigester 1:1 chromatographiert. Das langsamer laufende diastereomere Carbamat kann angereichert werden und durch Kristallisation aus Toluol rein erhalten werden (Schmp. 243-245°C). Durch Verseifung mit NaOH in EtOH bei 80°C erhält man das (+)-3,4-Dihydro-2,2-dimethyl-7-methoxy-6-phenylsulfonyl-trans-4-(2-oxo-1-pyrrolidinyl)-2H-benzo[b]pyran-3-ol mit dem Schmp.: 209-211°C und [α]_{D} = + 109° (c= 0,28; CHCl₃)

### Herstellung des Ausgangsmaterials:

3-Brom-3,4-dihydro-2,2-dimethyl-7-methoxy-6-phenylsulfonyl-2H-benzo[b]pyran-4-ol erhält man aus 2,2-Dimethyl-7-methoxy-6-phenylsulfonyl-2H-chromen und N-Bromsuccinimid in einem 9:1 Dimethylsulfoxid/H₂O Gemisch. Schmp.: 202 - 203°C.

2,2-Dimethyl-7-methoxy-6-phenylsulfonyl-2H-chromen erhält man aus 2,2-Dimethyl-4-hydroxy-7-methoxy-6-phenylsulfonylchromen mit Pyridin/Phosphoroxychlorid in Benzol.
Schmp: 140 - 41°C.

2,2-Dimethyl-4-hydroxy-7-methoxy-6-phenylsulfonylchroman erhält man aus 2,2-Dimethyl-7-methoxy-6-phenylsulfonylchroman-4-on mit Natriumborhydrid in Methanol.
Schmp.: 146 - 147°C.

2,2-Dimethyl-7-methoxy-6-phenylsulfonylchroman-4-on erhält man aus Phenylsulfonylchlorid, 2,2-Dimethyl-7-methoxychroman-4-on und Aluminiumchlorid in Methylenchlorid.
Schmp.: 223 - 25°C

### Beispiel 4

### 3,4-Dihydro-2,2-dimethyl-6-(4-methylphenylsulfonyl)-trans-4-(2 -oxo-1-pyrrolidinyl)-2H-benzo[b]pyran-3-ol

Zu 8,2 g (0,02 Mol) 3-Brom-3,4-dihydro-2,2-dimethyl-6-(4-methylphenylsulfonyl)-2H-benzo[b]pyran-4-ol in 30 ml Dimethylsulfoxid werden 0,75 g (0,025 Mol) 80 %iges NaH eingetragen. Nach einstündigem Rühren bei 20° werden weitere 0,75 g (0,025 Mol) 80 % NaH und 1,9 ml (0,025 Mol) 2-Pyrrolidon zugefügt und 45 Minuten bei 40° und 6 Stunden bei 20° gerührt. Nach Eintragen in Eiswasser wird der Niederschlag abgesaugt, getrocknet und mehrfach aus Methanol umkristallisiert.
Kristalle vom Schmp.: 242 - 243°C.

### Herstellung des Ausgangsmaterials

### 3-Brom-3,4-dihydro-2,2-dimethyl-6-(4-methylphenylsulfonyl)-2H-benzo[b]pyran-4-ol

Zu 12,6 g (0,04 Mol) 2,2-Dimethyl-6-(4-methylphenylsulfonyl)-chromen in einer Lösung aus 70 ml Dimethylsulfoxid und 1,4 ml Wasser werden unter Kühlung (Isopropanol/Trockeneis) bei ungefähr 15°C 14,2 g (0,08 Mol) frisch umkristallisiertes N-Bromsuccinimid eingetragen. Die Temperatur steigt vorübergehend auf 27°. Es wird auf 20°C abgekühlt und nach einstündigem Rühren in Eis/Essigester eingetragen. Die Essigesterphase wird mehrfach mit Wasser gewaschen und über Na₂SO₄ getrocknet. Beim Einengen kristallisiert das Bromhydrin-Derivat.
Kristalle vom Schmp.: 141 - 142°C.

### Beispiel 5

### 3,4-Dihydro-2,2-dimethyl-6-phenylsulfonyl-trans-4-(2-oxo-1-pyrrolidinyl)-2H-benzo[b]pyran-3-ol

Zu einer Suspension von 0,6 g (0,02 Mol) 80 %igem NaH in 10 ml DMSO wird eine Lösung aus 6,3 g (0,02 Mol) 3,4-Dihydro-2,2-dimethyl-3,4-epoxy-6-phenylsulfonyl-2H-benzo[b]pyran in 20 ml DMSO bei 20° zugetropft. Anschließend werden 2,3 ml (0,03 Mol) 2-Pyrrolidinon zugefügt und 1 Stunde bei 45° gerührt. Nach Stehen über nacht bei 20° wird in Eiswasser eingetragen. Der Niederschlag wird abgesaugt, neutral gewaschen, getrocknet und an Kieselgel mit Methylenchlorid/Methanol 19:1 chromatographiert. 30 ml-Fraktionen werden aufgefangen. Die Fraktionen 12-25 werden eingeengt und der Rückstand wird aus Acetonitril umkristallisiert.
Schmp.: 201-202°

### Herstellung des Ausgangsmaterials:

3,4-Dihydro-2,2-dimethyl-3,4-epoxy-6-phenylsulfonyl-2H-benzo[b]pyran erhalt man aus 3-Brom-3,4-dihydro-2,2-dimethyl-6-phenylsulfonyl-2H-benzo[b]pyran-4-ol mit NaH in DMSO.
Schmp.: 103-105°

3-Brom-3,4-dihydro-2,2-dimethyl-6-phenylsulfonyl-2H-benzo[b]pyran-4-ol erhalt man aus 2,2-Dimethyl-6-phenylsulfonyl-2H-chromen und N-Bromsuccinimid in einem 9:1 Dimethylsulfoxid/H₂O Gemisch
Schmp.: 126°

Das 2,2-Dimethyl-6-phenylsulfonyl-2H-chromen mit dem Schmp.: 70-71° wurde nach bekannten Methoden aus 4-Phenylsulfonylphenyl-1,1 -dimethylpropargylether hergestellt. Diesen Ether erhielt man in ebenfalls bekannter Weise aus 4-Phenylsulfonylphenol und 3-Methyl-3-chlorbutin.

(+)-3,4-Dihydro-2,2-dimethyl-6-phenylsulfonyl-4-(2-oxo-1-pyrrolidinyl)-2H-benzo[b]pyran-3-ol (Beispiel 5a)

(±)-3,4-Dihydro-2,2-dimethyl-6-phenylsulfonyl-4-(2-oxo-1-pyrroiidinyl)-2H-benzo[b]pyran-3-ol wird nach Standardmethoden mit (-)Menthoxyessigsäurechlorid verestert. Die diastereomeren Ester werden auf einer Kieselgelsäule mit Methylenchlorid/Essigester (9:1) getrennt und mit alkoholischer Natriumethylatlösung unter Rühren bei 20° verseift. Nach Verdünnen mit kaltem Wasser wird der Niederschlag abgesaugt und neutral gewaschen und mit Äther verrieben.
(+)-3,4-Dihydro-2,2-dimethyl-6-phenylsulfonyl-4-(2-oxo-1-pyrrolidinyl)-2H-benzo[b]pyran-3-ol
Schmp.: 122-123°[α]_{D} = + 39,5° (c= 1,Ethanol)

### Beispiel 6

### 6-(4-Chlorphenylsulfonyl)-3,4-dihydro-2,2-dimethyl-7-methoxy-trans-4-(2-oxo-1-pyrrolidinyl)-2H-benzo[b]pyran-3-ol

Die Verbindung wird analog Beispiel 1 aus 3-Brom-6-(4-Chlorphenylsulfonyl)-3,4-dihydro-2,2-dimethyl-7-methoxy-2H-benzo[b]pyran-4-ol hergestellt.
Weiße Kristalle mit Schmp.: 260-262°C

### Herstellung der Ausgangsverbindungen:

### Analog Beispiel 1:

3-Brom-6-(4-Chlorphenylsulfonyl)-3,4-dihydro-2,2-dimethyl-7-methoxy-2H-benzo[b]pyran-4-ol mit Schmp.: 175-177°C

6-(4-Chlorphenylsulfonyl)-2,2-dimethyl-7-methoxy-chromen mit Schmp.: 142-143°C

### Beispiel 7

### 6-(4-Bromphenylsulfonyl)-3,4-dihydro-2,2-dimethyl-7-methoxy-trans-4-(2-oxo-1-pyrrolidinyl)-2H-benzo[b]pyran-3-ol

Analog Beispiel 1 aus 3-Brom-6-(4-Bromphenylsulfonyl)-3,4-dihydro-2,2-dimethyl-7-methoxy-2H-benzo[b]pyran-4-ol.
Weiße Kristalle vom Schmp.: 281-282°C.

### Beispiel 8

### 3,4-Dihydro-2,2-dimethyl-7-methoxy-6-(4-methoxyphenylsulfonyl)-trans-4-(2-oxo-1-pyrrolidinyl)-2H-benzo[b]pyran-3-ol

Die Verbindung wird analog Beispiel 1 aus 3-Brom-3,4-dihydro-2,2-dimethyl-7-methoxy-6-(4-methoxyphenylsulfonyl)-2H-benzo[b]pyran-4-ol hergestellt und hat einen Schmp. von 286-287°C.

### Beispiel 9

### 3,4-Dihydro-2,2-dimethyl-7-methoxy-6-(2-thienylsulfonyl)-trans-4-(2-oxo-1-pyrrolidlnyl)-2H-benzo[b]pyran-3-ol

Analog Beispiel 1 aus 3-Brom-3,4-dihydro-2,2-dimethyl-7-methoxy-6-(2-thienylsulfonyl)-2H-benzo[b]pyran-4-ol,
Schmp.: 135-136°C.

### Beispiel 10

### 3,4-Dihydro-2,2-dimethyl-7-ethoxy-6-phenylsulfonyl-trans-4-(2-oxo-1-pyrrolidinyl)-2H-benzo[b]pyran-3-ol

Analog Beispiel 1 aus 3-Brom-3,4-dihydro-2,2-dimethyl-7-ethoxy-6-phenylsulfonyl-2H-benzo[b]pyran-4-ol,
Schmp: 197-198°C.

### Beispiel 11 .

### 3,4-Dihydro-2,2-dimethyl-7-methoxy-6-phenylsulfonyl-trans-4-(2-oxo-1-piperidinyl)-2H-benzo[b]pyran-3-ol

Analog Beispiel 2 aus 3-Brom-3,4-dihydro-2,2-dimethyl-7-methoxy-6-phenylsulfonyl-2H-benzo[b]pyran-4-ol,
Weiße Kristalle vom Schmp.: 157-158°C.

### Beispiel 12

### 6-(4-Cyanphenylsulfonyl)-3,4-dihydro-2,2-dimethyl-trans-4-(2-oxo-1-pyrrolidinyl)-2H-benzo[b]pyran-3-ol

Analog Beispiel 1 aus 3-Brom-6-(4-cyanphenylsulfonyl)-3,4-dihydro-2,2-dimethyl-2H-benzo[b]pyran-4-ol,
Weiße Kristalle vom Schmp.: 234-235°C.

### Herstellung des Ausgangsmaterials:

3-Brom-6-(4-cyanphenylsulfonyl)-3,4-dihydro-2,2-dimethyl-2H-benzo[b]pyran-4-ol erhält man wie unter Beispiel 3 beschrieben aus 6-(4-Cyanphenylsulfonyl)-2,2-dimethyl-3-chromen.
Schmp.: 157-158°C.

### Beispiel 13

### 3,4-Dihydro-2,2-dimethyl-6-(2-methoxyphenylsulfonyl)-trans-4-(2-oxo-1-pyrrolidinyl)-2H-benzo[b]pyran-3-ol

Analog Beispiel 1 aus 3-Brom-3,4-dihydro-2,2-dimethyl-6-(2-methoxyphenylsulfonyl)-2H-benzo[b]pyran-4-ol.
Weiße Kristalle vom Schmp.: 196-198°C.

### Beispiel 14

### 3,4-Dihydro-2,2-dimethyl-6-(2-methylphenylsulfonyl)-trans-4-(2-oxo-1-pyrrolidinyl)-2H-benzo[b]pyran-3-ol

Analog Beispiel 1. Weiße Kristalle vom Schmp.: 214-216°C.

### Beispiel 15

### 3,4-Dihydro-2,2-dimethyl-6-(2-chlorphenylsulfonyl)-trans-4-(2-oxo-1-pyrrolidinyl)-2H-benzo[b]pyran-3-ol

Analog Beispiel 1. Weiße Kristalle vom Schmp.: 85-87°C

### Beispiel 16

### Herstellung von 3,4-Dihydro-2,2-dimethyl-6-phenylsulfonyl-trans-4-(2-oxo-1-pyrrolidinyl)-2H-benzo[b]pyran-3-ol (Verbindung von Beispiel 5 nach Verfahrensvariante c)

Eine Lösung von 3-Brom-3,4-dihydro-2,2-dimethyl-6-phenylsulfonyl-2H-benzo[b]pyran-4-ol in Ethanol wird 8 Stunden bei 50° im Autoklaven bei einem Druck von 8 bar NH₃ geschüttelt. Nach dem Abkühlen wird bis zur Trockene eingeengt und aus Essigester umkristallisiert. Man erholt das 4-Amino-3,4-dihydro-2,2-dimethyl-6-phenylsulfonyl-2H-benzo[b]pyran-3-ol vom Schmp.: 160-163°C, das sofort einer Acylierung mit 4-Chlorbuttersäurechlorid unterworfen wird. Hierzu wird die Substanz sowie das Säurechlorid in CH₂Cl₂ und im Zweiphasengemisch mit 2N Natronlauge 24 Stunden bei Zimmertemperatur gerührt. Nach üblicher Aufarbeitung erhalt man das 4-(4-Chlorbutyrylamino)-3,4-dihydro-2,2-dimethyl-6-phenylsulfonyl-2H-benzo[b]pyran-4-ol vom Schmp.: 155-157°C. Die Cyclisierung zur Titelverbindung erfolgt durch Lösung der Substanz in Tetrahydrofuran, Zugabe einer stöchiometrischen Menge 80 %igem NaH-Suspension inÖl undRühren des Gemisches bei Zimmertemperatur über 24 Stunden. Das Endprodukt ist mit dem nach Verfahren b) gewonnenen Produkt identisch. Schmp.: 200-201°C. Wird das 4-Amino-3,4-dihydro-2,2-dimethyl-6-phenylsulfonyl-2H-benzo[b]pyran-3-ol einer Racematspaltung unterworfen, so kann aus seinem (+)-Enantiomer das reine (+)-Enantiomer aus Beispiel 5a mit den dort angegebenen Daten erhalten werden.

### Beispiel 17

### 3,4-Dihydro-2,2-dimethyl-6-phenylsulfoxy-trans-4-(2-oxo-1-pyrrolidinyl)-2H-benz[b]pyran-3-ol

Analog Beispiel 1. Schmp.: 211 - 212°C

### Pharmakologische Daten

### a) Beeinflussung von Atemwegsstörungen Einfluß auf die histamin-induzierte Bronchokonstriktion am Meerschweinchen

### Methode:

Albino-Meerschweinchen beiderlei Geschlechts mit einem Gewicht zwischen 450 und 550 g wurden mit 60 mg/kg Pentobarbital i.p. narkotisiert. Nach Tracheotomie wurden sie mit einer Starlingpumpe (Fa. Braun, Melsungen) beatmet. Das Atemvolumen wurde so gewählt, daß die Lungen ausgiebig belüftet wurden. Der Beatmungsdruck wurde mit Hilfe einer Wassersäule auf 80 mm H₂O eingestellt (Rosenthal und Dervinis). Die Atemfrequenz betrug 30 Atemzüge/min. Ein Überschuß an verfügbarer Atemluft kann durch einen Nebenweg im Einatemschlauch zu einer als Wasser-Überdruckventil geschalteten Waschflasche gelangen. Wird von der Starling-Pumpe durch den Einatemschlauch Luft in die Lunge geblasen, so füllt sich diese so lange, bis der Druck in dem System den durch die Höhe der Wassersäule eingestellten Wert (80 mm H₂O) erreicht hat. Die noch weiter zuströmende Luft fließt durch das Überdruckventil in die Waschflasche. Dieser Luftüberschuß, der nach Überschreiten des eingestellten Drucks in die Flasche entweicht, wird mit Hilfe eines Pistonrekorders gemessen und gilt als Maß für eine Veränderung des Atemwiderstands. Die Messung erfolgte in einer von der ursprünglichen Konzett-Rössler-Methode leicht modifizierten Weise, indem der Pistonrekorder durch ein Staudruckrohr nach Fleisch (Typ 0000) ersetzt wurde. Der auftretende Druckunterschled wurde mit einem Differenzdruckwandler Statham PM 97 TC erfaßt. Zur Aufzeichnung der Meßwerte diente ein Mehrfachkanalschreiber der Fa. Hellige.

Die Testsubstanzen werden als Aerosol mit Hilfe eines Ultraschallverneblers (Monaghan M 650) verabreicht. Die Verneblerkammer wird in den Einatemschlauch der Atempumpe zwischengeschaltet und den Tieren gestattet, das Aerosol 1 min lang einzuatmen. Das zu vernebelnde Volumen beträgt 0,02 ml/min.

Vernebelt wird eine physiologische Kochsalzlösung, der die zu prüfende Substanz in Form einer Lösung in Propandiol zugesetzt wird.

In einem Kontrollversuch wird sichergestellt, daß das Lösemittel Propandiol/physiologische Kochsalzlösung keine Eigenwirkung besitzt.

Eine Bronchokonstriktion wird durch Gaben von Histamindihydrochlorid (6 - 12 »g/kg i.v.) induziert. Die Injektion erfolgt durch einen in die V. jugularis eingeführten Katheter. Die Dosierung wird so gewählt, daß ein respiratorischer "Überfluß" von 60 % des angebotenen Atemvolumens während der Histamin-induzierten Bronchokonstriktion auftritt. Die Histamingaben erfolgen in 5-Minuten-Intervallen. Nach mindestens 3 gut reproduzierbaren Bronchokonstriktionen werden die Testsubstanzen als Aerosol 1 min lang verabreicht und nach einer Einwirkungszeit von 2 min erneut die Bronchokonstriktion induziert und in 5-Minuten-Intervallen wiederholt.

Der Grad der Hemmung der Histamin-induzierten Bronchokonstriktion nach Vorbehandlung mit der Testsubstanz wird als Maß für die bronchodilatatorische Wirksamkeit angesehen und als Veränderung in % gegenüber der Kontrolle angegeben.

Alle Ergebnisse werden der linearen Regression unterzogen und die ID₅₀ ermittelt.

### Ergebnisse:

| % Hemmung der Histamin-induzierten Bronchokonstriktion | | | |
|---|---|---|---|
| Verbindung | Dosis | n | % Hemmung |
| Beispiel 5a (+)-3,4-Dihydro-2,2-dimethyl-6-phenylsulfonyl-trans-4-(2-oxo-1-pyrrolidinyl)-2H-benzo[b]pyran-3-ol | 1 »g/kg | 6 | 14 ± 4 |
| | 3 »g/kg | 6 | 35 ± 4 |
| | 10 »g/kg | 6 | 77 ± 7 |
| ID₅₀: 4,39 »g/kg | | | |

## Patentansprüche

1. Verwendung einer Verbindung I 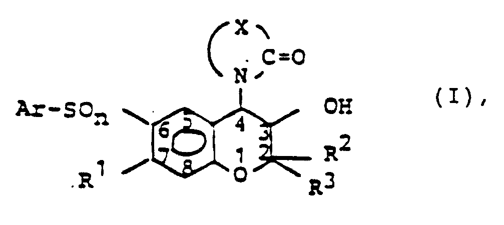 in welcher
R¹ für H, OH, (C₁-C₂)-Alkoxy, (C₁-C₂)-Alkyl oder NR⁴R⁵ steht, wobei R⁴ und R⁵ gleich oder verschieden sind und für H, (C₁-C₂)Alkyl oder (C₁-C₃)-Alkylcarbonyl stehen,
R²,R³ gleich oder verschieden sind und für Alkyl mit 1-4 C-Atomen stehen,
Ar für ein aromatisches oder heteroaromatisches System steht, das unsubstituiert oder durch 1 bis 3 gleiche oder verschiedene Reste (C₁-C₂)-Alkyl, (C₁-C₂)Alkoxy, Halogen, Trifluormethyl, CN, NO₂, CO-(C₁-C₂)Alkyl oder SOₘ-(C₁-C₂)-Alkyl mit m= 1 oder 2 substituiert ist,
n für 1 oder 2 steht,
X für eine Kette (CH₂)ᵣ steht, die durch ein Heteroatom 0, S oder NR⁶ unterbrochen sein kann, wobei R⁶ H oder (C₁-C₄)-Alkyl bedeutet und r für die Zahlen 2, 3, 4 oder 5 steht,
zur Herstellung eines Heilmittels gegen obstruktive Funktionsstörungen der Lunge.

2. Pharmazeutisches Präparat zur Behandlung von obstructiven Funktionsstörungen der Lunge, dadurch gekennzeichnet, daß es eine Verbindung I als Wirkstoff neben üblichen Zuschlagstoffen enthält.

3. Verbindung I zur Anwendung zur Behandlung von obstruktiven Funktionsstörungen der Lunge.

4. Verwendung einer Verbindung I nach Anspruch 1, bei der R¹ bis R³ und ArSOₙ wie in Anspruch 1 definiert sind, und X (CH₂)ᵣ mit r= 3 oder 4 bedeutet.

5. Verwendung nach Anspruch 1, dadurch gekennzeichnet, daß
R¹ bis R³ die oben erwähnten Bedeutungen haben,
Ar für unsubstituiertes oder wie nach Anspruch 1 substituiertes Phenyl steht, und
X (CH₂)ᵣ mit r= 3 oder 4 bedeutet.

6. Verwendung nach Anspruch 1, dadurch gekennzeichnet, daß
R¹ Wasserstoff bedeutet,
R² und R³ für (C₁-C₂)-Alkyl stehen,
Ar Phenyl bedeutet, das unsubstituiert oder durch (C₁-C₂)-Alkyl, (C₁-C₂)-Alkoxy oder Halogen einfach substituiert ist,
n 2 bedeutet, und
X (CH₂)ᵣ mit r= 3 oder 4 bedeutet.

7. Verwendung nach Anspruch 1, dadurch gekennzeichnet, daß
R¹ Wasserstoff bedeutet,
R² und R³ für (C₁-C₂)-Alkyl stehen,
Ar Phenyl bedeutet,
n 2 bedeutet, und
X (CH₂)₃ bedeutet.

8. Verwendung von 3,4-Dihydro-2,2-dimethyl-6-phenylsulfonyl-trans-4-(2-oxo-1-pyrrolidinyl)-2H-benzo[b]pyran-3-ol zur Herstellung eines Heilmittels gegen obstruktive Atemwegserkrankungen.

## Claims

1. The use of a compound I 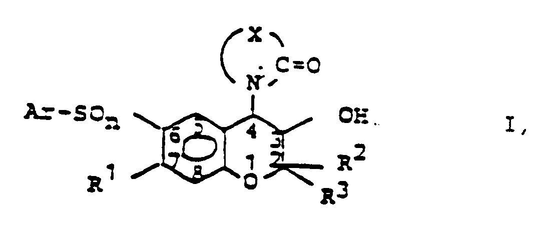 in which
R¹ represents H, OH, (C₁-C₂)-alkoxy, (C₁-C₂)-alkyl or NR⁴R⁵, where R⁴ and R⁵ are identical or different and represent H, (C₁-C₂)-alkyl or (C₁-C₃)-alkylcarbonyl,
R² and R³ are identical or different and represent alkyl having 1-4 carbon atoms,
Ar represents an aromatic or heteroaromatic system which is unsubstituted or substituted by 1 to 3 identical or different radicals (C₁-C₂)-alkyl, (C₁-C₂)-alkoxy, halogen, trifluoromethyl, CN, NO₂, CO-(C₁-C₂)-alkyl or SOₘ-(C₁-C₂)-alkyl with m = 1 or 2,
n represents 1 or 2,
X represents a chain (CH₂)ᵣ which can be interrupted by a heteroatom O, S or NR⁶, where R⁶ denotes H or (C₁-C₄)-alkyl, and r represents the numbers 2, 3, 4 or 5,
for the preparation of a remedy for obstructive functional disorders of the lungs.

2. A pharmaceutical product for the treatment of obstructive functional disorders of the lungs, which contains a compound I as active substance besides customary additives.

3. A compound I for use for the treatment of obstructive functional disorders of the lungs.

4. The use of a compound I as claimed in claim 1, in which
R¹ to R³ and ArSoₙ are as defined in claim 1, and X denotes (CH₂)ᵣ with r = 3 or 4.

5. The use as claimed in claim 1, wherein
R¹ to R³ have the abovementioned meanings,
Ar represents phenyl which is unsubstituted or substituted as in claim 1, and
X denotes (CH₂)ᵣ with r = 3 or 4.

6. The use as claimed in claim 1, wherein
R¹ denotes hydrogen,
R² and R³ represent (C₁-C₂)-alkyl,
Ar denotes phenyl which is unsubstituted or substituted once by (C₁-C₂)-alkyl, (C₁-C₂)-alkoxy or halogen,
n denotes 2, and (CH₂)ᵣ with r = 3 or 4.

7. The use as claimed in claim 1, wherein
R¹ denotes hydrogen,
R² and R³ represent (C₁-C₂)-alkyl,
Ar denotes phenyl,
n denotes 2, and
X denotes (CH₂)₃.

8. The use of 3,4-dihydro-2,2-dimethyl-6-phenylsulfonyl-trans-4-(2-oxo-1-pyrrolidinyl)-2H- benzo[b]pyran-3-ol for the preparation of a remedy for obstructive respiratory tract diseases.

## Revendications

1. Utilisation d'un composé I 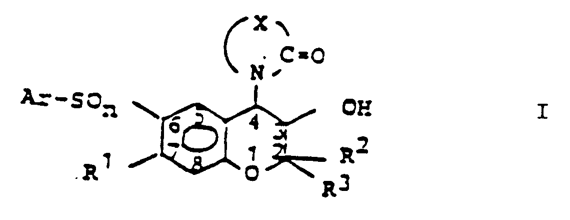 dans laquelle
R¹ représente H, OH, un groupe alcoxy en C₁-C₂, alkyle en C₁-C₂ ou NR⁴R⁵, R⁴ et R⁵ étant identiques ou différents et représentant H ou un groupe alkyle en C₁-C₂ ou alkyl(C₁-C₃)-carbonyle,
R², R³ sont identiques ou différents et représentent un groupe alkyle ayant de 1 à 4 atomes de carbone,
Ar représente un système aromatique ou hétéroaromatique qui est non substitué ou substitué par un 1 à 3 substituants, identiques ou différents, alkyle en C₁-C₂, alcoxy en C₁-C₂, halogène, trifluorométhyle, CN, NO₂, CO-alkyle(C₁-C₂) ou SOₘ-alkyle(C₁-C₂), m étant 1 ou 2,
n représente 1 ou 2,
X représente une chaîne (CH₂)ᵣ qui peut être interrompue par un hétéroatome O, S ou par NR⁶, R⁶ représentant H ou un groupe alkyle en C₁-C₄, et r représentant les nombres 2, 3, 4 ou 5,
pour la fabrication d'un médicament contre les troubles pulmonaires obstructifs.

2. Composition pharmaceutique pour le traitement de troubles pulmonaires obstructifs, caractérisée en ce qu'elle contient un composé I en tant que substance active, en plus d'additifs usuels.

3. Composé I pour utilisation dans le traitement de troubles pulmonaires obstructifs.

4. Utilisation d'un composé I selon la revendication 1, dans lequel R¹ à R³ et ArSOₙ sont tels que définis dans la revendication 1, et X représente (CH₂)ᵣ, avec r = 3 ou 4.

5. Utilisation selon la revendication 1, caractérisée en ce que
R¹ à R³ ont les significations mentionnées plus haut,
Ar représente un groupe phényle non substitué ou substitué selon la revendication 1, et
X représente (CH₂)ᵣ avec R = 3 ou 4.

6. Utilisation selon la revendication 1, caractérisée en ce que
R¹ représente un atome d'hydrogène,
R² et R³ représentent un groupe alkyle en C₁-C₂,
Ar représente un radical phényle qui est non substitué ou substitué une fois par un halogène ou par un groupe alkyle en C₁-C₂ ou alcoxy en C₁-C₂,
n est 2 et
X représente (CH₂)ᵣ avec r = 3 ou 4.

7. Utilisation selon la revendication 1, caractérisée en ce que
R¹ représente un atome d'hydrogène,
R² et R³ représentent un groupe alkyle en C₁-C₂,
Ar représente le groupe phényle,
n est 2 et
X représente (CH₂)₃.

8. Utilisation du 3,4-dihydro-2,2-diméthyl-6-phénylsulfonyl-*trans*-4-(2-oxo-1-pyrrolidinyl)-2H-benzo[b]pyranne-3-ol pour la fabrication d'un médicament contre des affections obstructives des voies respiratoires.
